(19) Europäisches Patentamt
European Patent Office
Office européen des brevets

(11) Veröffentlichungsnummer: **0 067 353**
**B1**

(12) # EUROPÄISCHE PATENTSCHRIFT

(45) Veröffentlichungstag der Patentschrift: **12.12.84**

(51) Int. Cl.³: **C 07 C 43/305,** C 07 C 41/50, C 07 D 317/20, C 07 D 319/06

(21) Anmeldenummer: **82104772.7**

(22) Anmeldetag: **01.06.82**

(54) **Verfahren zur Herstellung cyclischer Acetale.**

(30) Priorität: **13.06.81 DE 3123522**

(43) Veröffentlichungstag der Anmeldung: **22.12.82 Patentblatt 82/51**

(45) Bekanntmachung des Hinweises auf die Patenterteilung: **12.12.84 Patentblatt 84/50**

(84) Benannte Vertragsstaaten: **BE DE FR GB IT NL**

(56) Entgegenhaltungen: **DE - A - 2 548 911**

(73) Patentinhaber: **BAYER AG, Konzernverwaltung RP Patentabteilung, D-5090 Leverkusen 1 Bayerwerk (DE)**

(72) Erfinder: **Blazejak, Manfred, Dr., Richardstrasse 81, D-4000 Düsseldorf 1 (DE)**
Erfinder: **Grotkopp, Detlef, Dr., Cecilienallee 52, D-4000 Düsseldorf 1 (DE)**
Erfinder: **Haydn, Josef, Dr., Leipziger Strasse 27, D-5090 Leverkusen 1 (DE)**

**EP 0 067 353 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft ein Verfahren zur Herstellung cyclischer Acetale durch Umsetzung von Pentaerythrit mit Aldehyden in wäßrigem Medium.

Die DE-OS 2 548 911 beschreibt Kondensationsprodukte aus Tri-, Tetra-, Penta- und/oder Hexaolen mit Tetrahydro-$\Delta^3$-benzaldehyden oder Endomethylentetrahydro-$\Delta^3$-benzaldehyden im Molverhältnis 1 : 1 bis 1 : 3, in denen alle Aldehydgruppen vollständig acetalisiert vorliegen.

Die Verbindungen finden Verwendung als Ozonschutzmittel für Natur- und/oder Synthesekautschuk.

Die Kondensation der genannten Polyole mit den Tetrahydro-$\Delta^3$-benzaldehyden erfolgt, in dem man die Polyhydroxylverbindung mit dem Aldehyd in Gegenwart katalytischer Mengen eines sauren Dehydratisierungskatalysators bei Temperaturen von 0—200° C, umsetzt, wobei pro Mol Alkohol 1 bis 3 Mole Aldehyd eingesetzt werden.

Die Reaktion kann ohne Lösungsmittel oder in Gegenwart von Lösungsmitteln durchgeführt werden. Als Lösungsmittel sind sowohl polare als auch unpolare Lösungsmittel geeignet.

Führt man die Reaktion ohne Lösungsmittel, d. h. in der Schmelze durch, entsteht eine größere Anzahl harzartiger Nebenprodukte, die die Ausbeute an gewünschten Verbindungen stark verringern.

Setzt man organische Lösungsmittel ein, muß man diese reinigen und aufarbeiten, um sie aus wirtschaftlichen Gründen wieder einsetzen zu können. Dabei können zündfähige Lösungsmittelgemische entstehen sowie Emissionen von Lösungsmitteldämpfen eintreten. Aus ökologischer und sicherheitstechnischer Sicht sind dies Nachteile.

Es wurde nun gefunden, daß die Herstellung der genannten cyclischen Acetale auch durch Kondensation in Wasser ohne azeotrope Wasserdestillation bei erhöhten Temperaturen unter Druck und unter Zusatz eines sauren Katalysators durchgeführt werden kann. Das erfindungsgemäße Verfahren arbeitet ohne Zusatz von organischen Lösungsmitteln. Die Ausbeute ist quantitativ und das entsprechende Produkt geruchlos.

Die Erfindung betrifft daher ein Verfahren zur Herstellung von Kondensationsprodukten aus Pentaerythrit und Tetrahydro-$\Delta^3$-benzaldehydverbindungen der Formeln

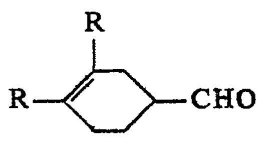 bzw. 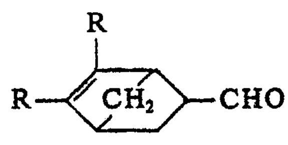

in denen R gleich oder verschieden Wasserstoff oder Methyl darstellt, im Molverhältnis 1 : 1 bis 1 : 3, wobei alle Aldehydgruppen acetalisiert vorliegen, in Gegenwart von katalytischen Mengen eines sauren Dehydratisierungskatalysators, dadurch gekennzeichnet, daß man die Reaktion im wäßrigen Medium unter Anwendung von Überdruck bei Temperaturen von 80—140° C durchführt.

Als saure Katalysatoren können die üblichen Mineralsäuren wie Salzsäure, Schwefelsäure, Phosphorsäure oder auch organische Säuren wie z. B. p-Toluolsulfonsäure oder auch Metallchloride wie z. B. Eisenchlorid oder Zinkchlorid oder Ionenaustauscher verwendet werden.

Die Katalysatoren werden bevorzugt in Mengen von 0,05 bis 5, besonders bevorzugt in Mengen von 0,1 bis 1 Gew.-% bezogen auf Aldehyd, eingesetzt.

Die einzusetzende Menge an Wasser ist nicht kritisch. Da das Wasser nach Reaktionsende abdestilliert werden muß, ist es unökonomisch, eine größere als notwendige Menge einzusetzen. Setzt man zu wenig Wasser ein, wird der Reaktionsansatz nicht mehr rührbar.

Es kann durchaus der Fall eintreten, daß die Kondensationsprodukte sich in Wasser nicht lösen. Für diesen Fall ist zu empfehlen, übliche Emulgatoren zuzusetzen. Allerdings ist es dann gegebenenfalls erforderlich, nach Reaktionsende übliche Trennmittel einzusetzen, die die Emulsion trennen.

Eine allgemeine Herstellungsweise sei wie folgt beschrieben:

Pentaerythrit, Wasser und Katalysator werden vorgegeben und bei der Siedetemperatur des Wassers der Tetrahydrobenzaldehyd zudosiert. Das Verfahren kann diskontinuierlich oder aber auch kontinuierlich z. B. in einer Kesselkaskade durchgeführt werden. Die Reaktionszeit kann variabel sein und wird in der Regel durch die technischen Vorrichtungen der Apparatur bestimmt. Ebenso kann auch die Entfernung des Wassers kontinuierlich z. B. in Dünnschichtern oder Fallstromverdampfern erfolgen. Durch eine analytische Bestimmung des nicht umgesetzten Pentaerythrits kann der Fortgang der Reaktion verfolgt werden. Der Endpunkt ist dann erreicht, wenn kein Pentaerythrit mehr in der Reaktionsmischung vorhanden ist. Danach wird das Wasser abgetrieben und der Rest Wasser im Vakuum entfernt.

Der verbleibende Rückstand bildet eine kristallin erstarrende Masse. Sie kann auf den bekannten Konfektioniermaschinen zu Pastillen oder Schneckengranulat verarbeitet werden. Eine besondere Variante ist die Möglichkeit, das flüssige Produkt auf ein anorganisches Trägermaterial wie z. B. Vulkasil in einer geeigneten Apparatur z. B. einen Horizontalmischer mit vertikaler Turboverteilung aufzuziehen, um so als feinverteiltes Material mit besonders aktiver Oberfläche verarbeitet werden zu

können. Dabei kann diese Oberfläche noch durch einen Wachsüberzug von Autoxidation geschützt werden.

Beispiel 1

| | |
|---|---|
| 350 Gew.-Teile | Pentaerythrit, |
| 8,4 Gew.-Teile | p-Toluolsulfonsäure, |
| 400 Gew.-Teile | Wasser, |
| 568 Gew.-Teile | Tetrahydrobenzaldehyd |

werden in einem Rührdruckbehälter gegeben und 6 Stunden auf 120° C erhitzt.

Am Manometer des Reaktionsbehälters wird dabei ein Überdruck von ca. 1 bar abgelesen.

Nach Abkühlung wird der Behälter geöffnet. Anschließend wird das Wasser unter Normaldruck bis zu einer Sumpftemperatur bis zu 130° C abdestilliert, der Rest wird im Vakuum entfernt. Der Destillationsrückstand kristallisiert beim Erkalten, er besteht aus dem gewünschten Produkt.

Die Ausbeute beträgt 804 Gew.-Teile entsprechend 97,6%, bezogen auf den Einsatz Pentaerythrit, Schmelzpunkt: 89,0—91,5° C.

**Patentanspruch**

Verfahren zur Herstellung von Kondensationsprodukten aus Pentaerythrit und Tetrahydro-$\Delta^3$-benzaldehydverbindungen der Formeln

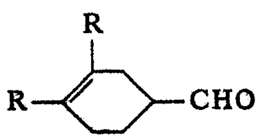 bzw. 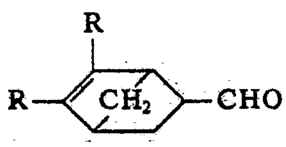

in denen R gleich oder verschieden Wasserstoff oder Methyl darstellt, im Molverhältnis 1 : 1 bis 1 : 3, wobei alle Aldehydgruppen acetalisiert vorliegen, in Gegenwart von katalytischen Mengen eines sauren Dehydratisierungskatalysators, dadurch gekennzeichnet, daß man die Reaktion in wäßrigem Medium unter Anwendung von Überdruck bei einer Temperatur von 80 bis 140° C durchführt.

**Claim**

Process for the preparation of condensation products of pentaerythritol and tetrahydro-$\Delta^3$-benzaldehyde compounds of the formulae

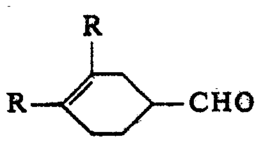 or 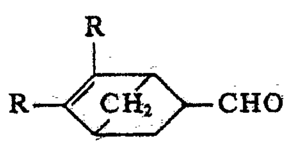

in which R ist identical or different and represents hydrogen or methyl, in a molar ratio of 1 : 1 to 1 : 3, all aldehyde groups being present in an acetalised form, in the presence of catalytic quantities of an acid dehydration catalyst, characterised in that the reaction is carried out in an aqueous medium using excess pressure at a temperature of 80 to 140° C.

**Revendication**

Procédé de préparation de produits de condensation du pentaérythritol et de tétrahydro-$\Delta^3$-benzaldéhydes de formules

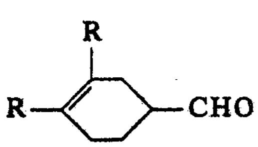 ou 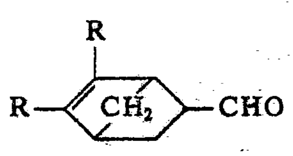

dans lesquelles R, qui peut avoir des significations identiques ou différentes, représente l'hydrogène ou le groupe méthyle, dans un rapport molaire de 1 : 1 à 1 : 3, tous les groupes aldéhyde étant à l'état acétalisé, en présence de quantités catalytiques d'un catalyseur de déshydratation acide, caractérisé en ce que l'on effectue la réaction en milieu aqueux sous pression à une température de 80 à 140° C.